Europäisches Patentamt

European Patent Office    (11) Publication number:    **0 017 681**

Office européen des brevets    **B1**

(19)

(12)    **EUROPEAN PATENT SPECIFICATION**

(45) Date of publication of patent specification: **25.05.83**    (51) Int. Cl.³: **C 07 C  53/02,**
**C 07 C  53/08,**
(21) Application number: **79300619.8**    **C 07 C  53/122,**
(22) Date of filing: **12.04.79**    **C 07 C  57/04, C 07 C  51/47**

(54) **Method for removing chromium ions from aqueous solutions of organic acids.**

| | |
|---|---|
| (43) Date of publication of application:<br>**29.10.80 Bulletin 80/22** | (73) Proprietor: **MITSUBISHI RAYON CO. LTD.**<br>**3-19, Kyobashi 2-chome Chuo-Ku**<br>**Tokyo 104 (JP)** |
| (45) Publication of the grant of the patent:<br>**25.05.83 Bulletin 83/21** | (72) Inventor: **Yoshioka, Teruhiko**<br>**No. 6-17, 7-chome Kuba**<br>**Otake City Hiroshima Pref. (JP)**<br>Inventor: **Matsuzawa, Hideo** |
| (84) Designated Contracting States:<br>**DE GB** | **No. 10-4, 2-chome Nishisakae**<br>**Otake City Hiroshima Pref. (JP)**<br>Inventor: **Okada, Kazuya**<br>**No. 2-3, 3-chome Kurokawa** |
| (56) References cited:<br>**FR - A - 1 290 212**<br><br>**Chemical Handbook of the Japanese Chemical**<br>**Association (1975), pp. 1629—1630** | **Otake City Hiroshima Pref. (JP)**<br>Inventor: **Ikeda, Minoru**<br>**No. 908-224, Ono-cho**<br>**Saiki-gun Hiroshima Pref. (JP)** |
| | (74) Representative: **Warden, John Christopher et al,**<br>**R.G.C. Jenkins & Co. 12-15, Fetter Lane**<br>**London EC4A 1PL (GB)** |

Courier Press, Leamington Spa, England.

**0017681**

### Method for removing chromium ions from aqueous solutions of organic acids

This invention relates to a method of efficiently removing chromium ions from an aqueous solution of an organic acid containing chromium.

An aqueous solution of an organic acid is often handled industrially using apparatus made of a stainless steel.

Examples include the recovery from aqueous solutions of acetic acid, acrylic acid or methacrylic acid by extracting and distilling operations and the production of tertiary butyl alcohol by reacting isobutylene with water containing an organic acid as mentioned in Japanese Published Patent Applications Nos. 32116 and 126603/1975.

In such cases, depending on the operating conditions, metals such as iron, nickel and chromium may dissolve out slightly due to corrosion of the stainless steel material.

Occasionally it is necessary, in order to prevent poisoning the catalyst, or for other reasons, to remove these metals. For example, in the method of the abovementioned Japanese Published Patent Application Nos 32116 and 126603/1975, metallic ions in a circulating aqueous solution of an organic acid are removed.

French Patent Specification 1,290,212 describes a process for decolourising organic solutions of vegetable origin and containing ions of calcium and iron mixed with amino acids and other coloured impurities, by ion exchange with strong acid groups at temperatures over 60°C. Chromium ions are not discussed. The process avoids the need for using active carbon for decolourisation. The present inventors have attempted to remove chromium ions by ion exchange but have unexpectedly encountered the problem that iron and nickel can be removed but that chromium is very difficult to remove. We have discovered that where the organic acid is at 100% concentration, chromium ions are comparatively well ion-exchanged but, in the case of an aqueous solution of an organic acid, chromium are very difficult to ion-exchange, and furthermore the ease or difficulty of the ion exchange of chromium ion depends remarkably on the temperature: the higher the temperature, the easier the ion exchange.

This fact is hereafter explained in the case of acetic acid.

(1) Variation of chromium ion removal with the acetic acid concentration. Aqueous solutions of acetic acid of various concentrations containing chromium acetate (containing 595 ppm as chromium) and an acetic acid solution were prepared and were passed at S.V.[1] (space velocity)=2 hr.$^{-1}$ at a room temperature (27°C) through a layer charged with 5 ml of a strong acid cationic exchange resin (IR-200)[2]. The results are shown in Table 1.

[1]S.V.=volume of aqueous solution/(volume of resin × hours).
[2]One grade of "Amberlite" (Registered Trade Mark of Rohm and Haas). It contains sulphuric acid groups attached to a styrene-divinylbenzene matrix.

TABLE 1

| Acetic acid concentration (%) by weight | 0 10 30 50 70 85 | 95 | 100 |
|---|---|---|---|
| Amount of the solution passed until chromium ion began to leak (ml) | about 10 | about 110 | about 180 |

The theoretically ion-exchangeable amount of the solution was about 220 ml.

It is evident from these results that, in the case of an aqueous solution of acetic acid of concentration less than 85%, the leakage of chromium ions can be recognized immediately; in the case of 100% acetic acid, the ion exchange will be possible up to 180 ml, near the theoretical amount of 220 ml and, in the case of an aqueous solution of 95% acetic acid, the ion exchange will be intermediate between them.

It is thus recognized that, in the case of an aqueous solution of 95% acetic acid at most, chromium ions are very difficult to ion-exchange.

(2) Variation of chromium ion removal with temperature. An aqueous solution of 85% acetic acid containing chromium acetate (containing 595 ppm as of chromium) was passed at S.V.=4 at various temperatures through a layer charged with 5 ml of a strong acid cation exchange resin (IR-200).

The results of measuring the amounts of the solution passed until chromium ion began to leak are shown in Table 2.

2

**0017681**

TABLE 2

| Temperature (°C) | 30 | 40 | 50 | 55 | 60 | 70 | 80 |
|---|---|---|---|---|---|---|---|
| Amount of the solution passed until chromium ion began to leak (ml) | about 10 | 20 | 30 | 90 | 110 | 150 | 210 |

It is found from these results that the ease or difficulty of the ion exchange of chromium ions is remarkably influenced by temperature and that at 55°C or above, the effect is particularly high. At 80°C, it is possible to exchange substantially up to the exchange capacity (about 220 ml) of the solution. This discovery makes it now possible to remove chromium ions from an aqueous solution of an organic acid with an ion exchange resin.

When the removal of chromium ions (contained at 500 ppm) from an aqueous solution of 85% methacrylic acid using methacrylic acid as an organic acid was attempted by the same method, at the room temperature (25°C), it was found that with the passage of 10 ml of the aqueous solution, 108 ppm of chromium leaked but, at 55°C, only 24 ppm leaked and, at 80°C, only 2 ppm at most leaked.

Although the temperature effect is seen to be somewhat different depending on the kind of the organic acid, we have found that in aqueous solutions of such other acids as acrylic acid, formic acid or propionic acid, at the room temperature chromium ions are only slightly ion-exchanged, but at 55°C or above or particularly at or above 70 to 80°C, the velocity of the chromium ion exchange becomes remarkably high, the exchanged amount is large and the chromium removing effect is high.

As evident from the above explanation, the present invention is characterized by treating and removing chromium ions from an aqueous solution of an organic acid of at most 95% concentration at a temperature of 55°C or above, using an ion exchange resin.

In the present invention, the chromium removing effect can be recognized at a treating temperature of 55°C or above but the higher the temperature, the more remarkable is the effect and particularly, above 70°C, the effect is high.

The aqueous solution of the organic acid of the present invention is of an organic acid concentration of 95% at most and the effect is high in removing chromium in an aqueous solution of an organic acid of a concentration particularly of 85% at most.

The invention is hereafter further explained by reference to the following Examples.

Examples 1 to 3

An aqueous solution of 85% acetic acid containing chromium acetate (containing 595 ppm as of chromium) was passed at S.V.=4 at a temperature of each of 55, 70 and 80°C through a layer charged with 5 ml of a strong acid cation exchange resin (IR-200).

The amounts of the solution passed until chromium ion began to leak are shown in Table 3.

Controls 1 to 3

The experimental results of making the same experiments as in Examples 1 to 3 at temperatures of 30, 40 and 50°C are also shown in Table 3.

TABLE 3

| | Temperature (°C) | Amount of the solution passed until chromium ion began to leak (ml) |
|---|---|---|
| Example 1 | 80 | about 210 |
| 2 | 70 | 150 |
| 3 | 55 | 90 |
| Control 1 | 50 | about 30 |
| 2 | 40 | 20 |
| 3 | 30 | 10 |

As evident from the abovementioned Examples and Controls, at a temperature of 55°C or above it is easy to remove chromium ions.

3

Examples 4 to 7

An aqueous solution of acetic acid of an acetic acid concentration of each of 10, 50, 70 and 95% containing chromium acetate (595 ppm as of chromium) was passed at S.V.=2 at 80°C through a layer charged with 5 ml of IR-200.

The results are shown in Table 4.

Controls 4 to 7

The same experiments as in Examples 4 to 7 were made at a temperature of 27°C.
The results are shown also in Table 4.

TABLE 4

| | Acetic acid concentration | Temperature | Amount of the solution passed until chromium ion began to leak |
|---|---|---|---|
| Example 4 | 10% | 80°C | about 210 ml |
| 5 | 50 | 80 | 210 |
| 6 | 70 | 80 | 210 |
| 7 | 95 | 80 | 210 |
| Control 4 | 10% | 27°C | about 10 ml |
| 5 | 50 | 27 | 10 |
| 6 | 70 | 27 | 10 |
| 7 | 95 | 27 | 110 |

As evident from the abovementioned Examples and Controls, even if at a variety of different concentrations of acid, the effect of the present invention can be recognized.

Examples 8 to 11

A chromium salt of each of formic acid, propionic acid, acrylic acid and methacrylic acid was dissolved in an aqueous solution of 85% of the corresponding organic acid (containing a concentration of 595 ppm of chromium).

This solution was passed at S.V.=4 at a temperature of 80°C through a layer charged with 5 ml of IR-200.

The results are shown in Table 5.

Controls 8 to 11

The same experiments as in Examples 8 to 11 were made at a temperature of 27°C.
The results are shown also in Table 5.

TABLE 5

| | Organic acid | Temperature | Amount of the solution passed until chromium ion began to leak |
|---|---|---|---|
| Example 8 | Formic acid | 80°C | about 210 ml |
| 9 | Propionic acid | 80 | |
| 10 | Acrylic acid | 80 | |
| 11 | Methacrylic acid | 80 | |
| Control 8 | Formic acid | 27°C | 10 ml at most |
| 9 | Propionic acid | 27 | |
| 10 | Acrylic acid | 27 | |
| 11 | Methacrylic acid | 27 | |

It is found from the abovementioned Examples and Controls that, even with a variety of organic acids, the effect of the present invention is remarkable.

**0017681**

**Claims**

1. A method of removing chromium ions from an aqueous solution of an organic acid of concentration not more than 95% (by weight), containing chromium ions, characterised by an ion exchanging process carried out at a temperature of 55°C or above using a cation exchange resin.

2. A method according to claim 1 characterised in that the organic acid comprises at least one selected from among formic acid, acetic acid, propionic acid, acrylic acid and methacrylic acid.

3. A method according to claim 1 characterised in that the acid is acetic acid.

4. A method according to any of claims 1 to 3 characterised in that the aqueous solution is of concentration not more than 85% (by weight).

5. A method according to any preceding claim characterised in that the temperature is at 70°C or above.

**Patentansprüche**

1. Verfahren zur Entfernung von Chromionen aus einer Chromionen enthaltenden, wäßrigen Lösung einer organichen Säure mit einer Konzentration von nicht mehr als 95 Gew.-%, gekennzeichnet durch ein Ionenaustauschverfahren, das bei einer Temperatur von 55°C oder darüber unter Anwendung eines Kationenaustauscherharzes durchgeführt wird.

2. Verfahren nach Anspruch 1, dadurch gekennzeichnet, daß die organische Säure aus mindestens einer Säure besteht, die aus Ameisensäure, Essigsäure, Propionsäure, Acrylsäure und Methacrylsäure ausgewählt ist.

3. Verfahren nach Anspruch 1, dadurch gekennzeichnet, daß die Säure Essigsäure ist.

4. Verfahren nach einem der Ansprüche 1 bis 3, dadurch gekennzeichnet, daß die wäßrige Lösung eine Konzentration von nicht mehr als 85 Gew.-% hat.

5. Verfahren nach einem der vorhergehenden Ansprüche, dadurch gekennzeichnet, daß die Temperatur bei 70°C oder darüber liegt.

**Revendications**

1. Procédé d'élimination des ions chrome d'une solution aqueuse d'un acids organique dont la concentration ne dépasse pas 95% (en poids), contenant des ions chrome, caractérisé par un procédé d'échange d'ions exécuté à une température au moins égale à 55°C avec utilisation d'une résine échangeuse de cations.

2. Procédé selon la revendication 1, caractérisé en ce que l'acide organique est constitué par au moins un acide choisi parmi l'acide formique, l'acide acétique, l'acide propionique, l'acide acrylique et l'acide méthacrylique.

3. Procédé selon la revendication 1, caractérisé en ce que l'acide est l'acide acétique.

4. Procédé selon l'une quelconque des revendications 1 à 3, caractérisé en ce que la solution aqueuse est d'une concentration ne dépassant pas 85% (en poids).

5. Procédé selon l'une quelconque des revendications précédentes, caractérisé en ce que la température est au moins égale à 70°C.